# EUROPEAN PATENT SPECIFICATION

(11) **EP 4 699 529 B1**
(45) Date of publication and mention of the grant of the patent: **11.11.2026**
(21) Application number: 25196908.5
(22) Date of filing: 20.08.2025
(51) Int. Cl.: A61B 5/06, A61B 5/00, A61B 5/339, A61B 18/14, A61B 34/20, G06T 19/20

(54) **CATHETER DEPTH PERCEPTION TOOLS ON MAP**
KATHETERTIEFENWAHRNEHMUNGSWERKZEUGE AUF EINER KARTE
OUTILS DE PERCEPTION DE PROFONDEUR DE CATHÉTER SUR UNE CARTE

(30) Priority: 21.08.2024 US 202463685463 P; 09.07.2025 US 202519263573
(43) Date of publication of application: 25.02.2026
(73) Proprietor: Biosense Webster (Israel) Ltd., 2066717 Yokneam (IL)
(72) Inventor: SEGEV, Meytal, 2066717 Yokneam (IL); ZOABI, Akram, 2066717 Yokneam (IL); MASSARWA, Fady, 2066717 Yokneam (IL); ALTMAN, Sigal, 2066717 Yokneam (IL); MATALON, Naor Avidor, 2066717 Yokneam (IL)
(74) Representative: Carpmaels & Ransford LLP

(56) References cited:
- US-A1- 2015 094 568
- US-A1- 2022 160 251
- US-A1- 20240 212 135

## Description

### CROSS-REFERENCE TO RELATED APPLICATIONS

This application claims the benefit of U.S. Provisional Patent Application 63/685,463, filed August 21, 2024.

### FIELD OF THE DISCLOSURE

This disclosure relates generally to the analysis of electroanatomical (EA) maps, and specifically to a system and method for representing catheter depth on a map.

### BACKGROUND OF THE DISCLOSURE

A 3D EA map of a cardiac chamber is typically presented on a 2D display, which may make it difficult for a physician surveying the map to understand the location of a catheter's distal end assembly relative to the chamber walls.

US2022/0160251 A1 discloses a system for electrophysiological measurement including a probe having a distal end configured for insertion into a body cavity of a patient and including electrodes that are disposed along the distal end and are configured to contact tissue at multiple locations within the body cavity while an operator manipulates the probe. A processor is configured to acquire electrophysiological (EP) signals from the electrodes within the body cavity, to apply one or more filtering criteria to the EP signals in order to select a first set of the EP signals while rejecting a second set of the EP signals, to render an image to a display based on the EP signals in the first set, and to output to the operator an indication of a reason for the rejection of the EP signals in the second set.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electrophysiology (EP) pacing, EA mapping, and ablation system, according to an example of the present disclosure;
Fig. 2 is an EA map (in orthographic view) of a left atrium with catheter depth perception tools incorporated, according to an example of the present disclosure; and
Fig. 3 shows a depth perception achieved with a visualization technique of light and shade applied to a 2D object overlaid on an anatomical map, according to an example of the present disclosure.

### DETAILED DESCRIPTION OF EXAMPLES

### OVERVIEW

Cardiac electro-anatomical (EA) catheter mapping is a technique that produces a three-dimensional (3D) EA map of the inner surface of cardiac chamber wall tissue, such as a late activation time (LAT) map of a left atrium. A physician can use the EA map to determine the precise location of an arrhythmia source or, when performing a medical procedure such as cardiac ablation, determine where to apply treatment (e.g., isolation).

A 3D EA map of a cardiac chamber is typically presented on a 2D display as an orthographic projection. Orthographic projection gives an accurate rendering of the cardiac chamber and devices (e.g., catheters) inside. A physician may rotate the map on the display and view the map from any viewing direction in space.

Orthographic projection, however, provides no sense of depth as the distance from the virtual viewer to an object does not affect the size of the rendered object. As a result, a physician viewing the EA map on a display may find it difficult to understand the current location of a catheter's distal end assembly relative to the chamber walls.

Examples of the present disclosure described herein provide catheter depth perception tools that let the physician utilize the advantages of an orthographically projected map together with being given a clear visual indication of the catheter's distal end assembly relative to the chamber walls at any given viewing direction.

In this disclosure, a processor defines a viewing direction of the 3D map as a ray superimposed on a vector from a virtual viewer to a catheter reference point location (e.g., to a catheter's landmark location such as a distal edge location of a shaft of the catheter). The processor receives the position information of the catheter located within the cardiac chamber, from a catheter positioning system.

Any given viewing direction of the 3D map (i.e., the direction of the orthograph projection) defines this way a ray that crosses the near/proximal (e.g., front-side) wall tissue and the remote/distal (e.g., back-side) wall tissue of the cardiac chamber. The crossing locations are defined per that view, respectively, as a proximal point location and a distal point location. The unique ray of the virtual viewer also crosses a catheter reference point location. The processor calculates the location over the ray of the reference point relative to the cardiac chamber view-dependent distance, based on the position information.

This way, the catheter's reference location (e.g., in the depth dimension) can be presented (e.g., on a scale) relative to the proximal wall and distal wall point locations.

In one example, the disclosed technique provides a view-dependent tool on the displayed map comprising a scale of catheter depth that is visualized in two ways:
1.A min-max dimension scale "per given view" near a transparent additional optional view (also called herein auxiliary map) that can be adjusted to a superior view.
2. Visualization overlaid of the catheter's shaft, of its location (e.g., color bar automatically adjusted based on proximity to the proximal point location).

These two visualizations provide a physician real-time sense of the depth, with the actual relative depth value on the scale of the chosen view.

In an example, for any given view direction, a processor presents an auxiliary orthographic view of the EA map (i.e., presents an auxiliary map) as seen from a direction orthogonal to the ray's direction (e.g., a superior view). The auxiliary map shows the catheter's location relative to the distal and proximal point locations along the viewing direction.

In addition, a scale located by the side of the auxiliary map gives the estimated distance of the catheter from the proximal wall relative to the total distance between the distal and proximal walls.

A similar scale can be superimposed on the catheter's shaft so that the physician can focus on the catheter and still receive the value of its depth in the chamber.

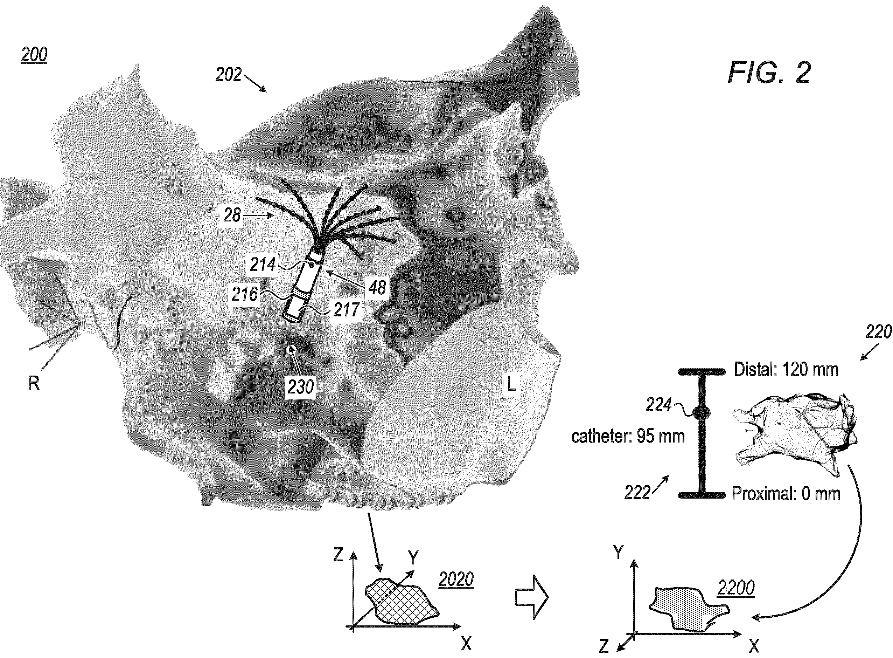

Further to that, the catheter's relative depth is indicated by being graphically encoded; for example, a distal portion of the shaft's color may vary from one color to another depending on how close the catheter is to the proximal and distal point locations. In a specific implementation seen in Fig. 2, the red portion appears larger as the catheter is closer to the distal wall (along the chamber proximal-distal wall distance defined by the viewing ray) and the green portion appears larger as it nears the proximal wall.

In another example, the technique provides depth perception for catheter navigation using light and shadow effects. A processor runs an algorithm that manipulates the catheter's perceived illumination such that the catheter's appearance changes based on its proximity to the viewer. Specifically, the catheter appears to be more illuminated when it is closer (i.e., proximal) to the viewer and more shaded when it is further away (i.e., distal). This visual cue gives the user a sense of the catheter's depth.

In yet another example, a processor colors the catheter image itself or a legend attached to the catheter. The catheter or the attached legend may be colored in one way (e.g., red) if the catheter is closer to the distal wall, while colored in another way (e.g., green) if the catheter is closer to the proximal wall.

Finally, the disclosed tool can also be applied or assist in veins anatomy perception to enhance optimization of ablating catheter location to improve electrical ablation.

The disclosed technique allows a physician to easily and intuitively view and comprehend the catheter's location in the depth dimension in the 3D maps using several unique visual presentations.

### SYSTEM DESCRIPTION

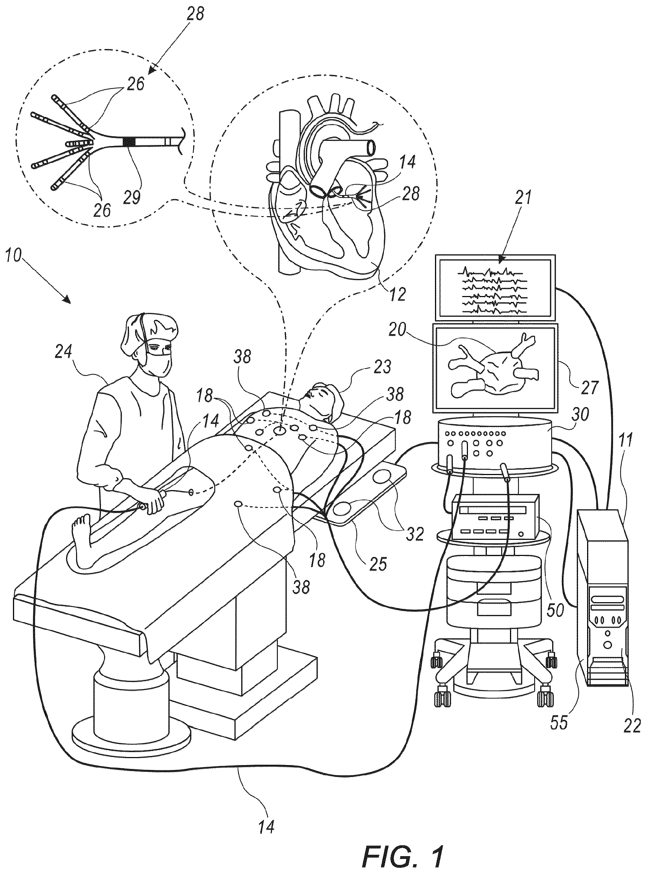

Fig. 1 is a schematic, pictorial illustration of a catheter-based electrophysiology (EP) pacing, EA mapping, and ablation system 10, according to an example of the present disclosure.

System 10 includes multiple catheters, which are percutaneously inserted by a physician 24 through the patient's vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated to sensing Intracardiac Electrogram (IEGM) signals and/or mapping heart 12, catheters dedicated to ablating, and/or catheters dedicated to both sensing and/or mapping and ablating. An example catheter 14 that is configured for sensing and mapping is illustrated herein. Physician 24 brings a distal end assembly 28 of catheter 14 into contact with the heart wall for sensing electro-potentials at a target site in heart 12, as well as for mapping one or more chambers of the heart, such as the left or right atriums.

Catheter 14 is an exemplary multi-spine catheter that includes multiple electrodes 26 distributed on the spines of the catheter. Catheter 14 may additionally include a position sensor 29 embedded in or near distal end assembly 28 for tracking the position and orientation of distal end assembly 28. Optionally and preferably, position sensor 29 is a magnetic based position sensor including three magnetic coils for sensing three-dimensional (3D) position and orientation.

A magnetic-based position sensor 29 may be operated together with a location pad 25, including a plurality of magnetic coils 32 configured to generate magnetic fields in a predefined working volume. The real-time position of distal end assembly 28 of catheter 14 may be tracked based on magnetic fields generated with location pad 25 and sensed by magnetic-based position sensor 29. Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on a patient 23 to establish a location reference for location pad 25 as well as impedance-based tracking of at least some electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described **in** U.S. Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) that may be captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 26. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, a power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, a processor 22 with memory or storage with appropriate operating software loaded therein, and user interface capability. Workstation 55 may provide multiple functions, optionally including (1) modeling the endocardial anatomy in three-dimensions (3D) and rendering a model or anatomical map 20 of all or part of heart 12 for display on a display device 27, typically by first mapping points of a selected portion of the heart, forming a mesh of the points, then covering the mesh with a surface, (2) displaying on display device 27 activation sequences (or other data) compiled from recorded electrograms 21 in representative visual indicia or imagery superimposed on the rendered anatomical map 20, (3) displaying real-time location and orientation of distal end assembly 28 within the heart chamber, and (4) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

In general, processor 22 may be embodied as a single processor, or as a cooperatively networked or clustered set of processors. The functionality of the processor may be implemented solely in hardware, e.g., using one or more fixed-function or general-purpose integrated circuits, Application-Specific Integrated Circuits (ASICs), and/or Field-Programmable Gate Arrays (FPGAs). Alternatively, this functionality may be implemented at least partly in software. For example, the processor may be embodied as a programmed processor comprising, for example, a central processing unit (CPU) and/or a Graphics Processing Unit (GPU). Program code, including software programs and/or data, may be loaded for execution and processing by the CPU and/or GPU. The program code and/or data may be downloaded to the processor electronically, over a network, for example. Alternatively, or additionally, the program code and/or data may be provided and/or stored on non-transitory tangible media, such as magnetic, optical, or electronic memory. Such program code and/or data, when provided to the processor, produce a machine or special-purpose computer, configured to perform the tasks described herein.

### CATHETER DEPTH PERCEPTION TOOLS ON MAP

Fig. 2 is an EA map 200 (viewed as an orthographic projection 202) of a left atrium with catheter depth perception tools (220, 222, 230) incorporated, according to an example of the present disclosure. Fig. 2 shows a multi-spine, multi-electrode, distal end assembly 28 catheter inside the cardiac chamber. The physician cannot know how posterior or anterior the multi-electrode assembly 28 is in any given view.

As many modern catheter assemblies (e.g., multi-spine, basket, lasso) have extended shapes over space, trying to indicate the location of individual elements (e.g., electrodes, spines) of the assembly may result in visual clutter.

To mitigate this, the disclosed technique defines a catheter reference point location to represent the assembly. One such reference point location can be location 214 on the distal edge of the catheter shaft 48. Another reference point location may be the center of a volume of revolution defined by the assembly (e.g., the center of an inner volume of a basket). The position tracking system continuously calculates the catheter's location during the EA mapping, as described in Fig. 1, thereby making the reference point location value known per each view.

The disclosed technique provides a distance scale of 222 of catheter depth that shows a representation (224) of the catheter's location 214 along the distance scale 222, relative to the cardiac chamber's view-dependent distal and proximal wall point locations.

To ease the physician's workflow, the catheter's view-dependent depth location 214 is also shown (230) near shaft 48. The current catheter position of 95 mm over a 120 mm view-dependent distance 222 between the cardiac walls is much more distal than proximal. To visually reflect this distal position, the disclosed technique colors (217) a catheter shaft 48 portion 216 near assembly 28 in a first color (e.g., red).

If the operator moves the catheter proximally so that the catheter's location 214 value becomes smaller than 60 mm, portion 216 (e.g., bar 216's) color 217 will change to a second color (e.g., green).

In the shown example, the processor presents a view-dependent auxiliary orthographic view 220 of map 200 from a superior view. Transparent auxiliary map 220 is received as an orthographic projection of the 3D EA map from a 90-degree rotated X-Y view 2200 of the X-Z view 2020 of orthographic projection 202 of map 202. Auxiliary map 220 shows the catheter's location relative to the distal and proximal cardiac chamber walls along the original viewing direction used in orthographic projection 202 of map 200.

### CATHETER DEPTH PERCEPTION BY SHADING

As noted above, the disclosed technique may provide depth perception for catheter navigation using light and shadow effects. In this example, a processor runs an algorithm that manipulates perceived lighting on the catheter such that the catheter's image appearance changes based on its proximity to the viewer. Specifically, the catheter appears to be more illuminated when it is closer (i.e., proximal) to the viewer and darker when it is farther away (i.e., distal). This visual cue may give the user a sense of the catheter's depth.

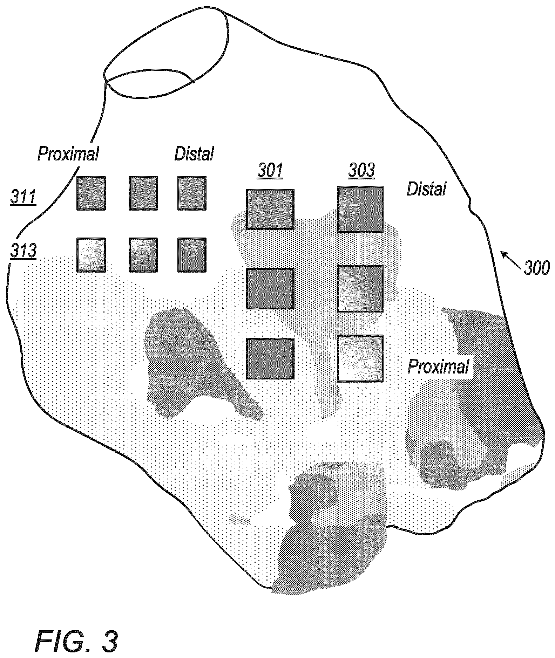

Fig. 3 shows a depth perception achieved with a visualization technique of light and shade applied to a 2D object overlaid on an anatomical map, according to an example of the present disclosure.

As seen in column 301 and row 311 of the 2D object, no sense of depth is achieved wherever the depth of the 2D object is assumed to be (i.e., from proximally to distally as seen by virtual viewer).

Column 303 and row 313, on the other hand, demonstrate how manipulating the light and shade of the 2D object visualization yields a sense of the object's depth. For example, if the 2D object is overlayed on a catheter, the catheter would appear to be more illuminated when it is closer to the viewer and appear more shaded when it is farther away. In other words, the illumination and shading of the 2D object would be determined by a virtual light-source (not shown) placed above the portion of the 3D map more proximal to the viewer.

The shaded 2D object may be a legend attached to the catheter or be the catheter image itself.

It will be appreciated that the present disclosure is not limited to what has been particularly shown and described hereinabove. Rather, the scope of the invention is defined by the claims and includes both combinations and sub-combinations of the various features described hereinabove, as well as variations and modifications thereof which would occur to persons skilled in the art upon reading the foregoing description and which fall within the scope of the claims.

## Claims

1. A system (10), comprising:
a display device (27); and
a processor (22), which is configured to:
receive an electroanatomical (EA) 3D map (20, 200) of a cardiac chamber, the map configured to be displayed on the display device as a view-dependent (2200, 2020) orthographic projection (202);
receive position information of a catheter (14) located within the cardiac chamber, from a catheter positioning system;
define a reference point location (214) of the catheter displayed on the 3D map;
for any given viewing direction of the 3D map, define the viewing direction as a ray superimposed on a vector from a virtual viewer to the reference point location;
calculate a view-dependent cardiac chamber distance (222) between a distal wall location along the ray and a proximal wall location along the ray;
calculate a location over the ray of the reference point location (214) relative to the cardiac chamber view-dependent distance (222), based on the position information; and
indicate (217, 224, 303) the calculated reference point location (214) relative to the cardiac chamber view-dependent distance (222) to a user.

2. The system (10) according to claim 1, wherein the processor (22) is configured to indicate the calculated reference point location (214) by displaying the calculated location value over a numerical scale defined by the cardiac chamber view-dependent (2200, 2020) distance (222).

3. The system (10) according to claim 1, wherein the processor (22) is configured to indicate the calculated reference point location (214) by displaying an auxiliary map (220) obtained from an orthographic view from a direction orthogonal to the given viewing direction.

4. The system (10) according to claim 1, wherein the processor (22) is configured to indicate the calculated reference point location (214) by graphically encoding (217) a portion (216) of the catheter (14) according to the calculated reference location value (224) over a numerical scale defined by the cardiac chamber view-dependent distance (222).

5. The system (10) according to claim 4, wherein the processor (22) is configured to graphically encode (217) the portion (216) of the catheter (14) by a first color if the calculated reference location value is smaller than half the cardiac chamber view-dependent distance (222) and by a second color if the calculated reference location value is higher than half the cardiac chamber view-dependent distance (222) .

6. The system (10) according to any of claims 1 and 5, wherein the processor (22) is configured to indicate the calculated reference point location (214) by displaying at least a portion (28, 48) of the catheter as illuminated differently (303, 313) according to its view-dependent distance (222) from the user.

7. The system (10) according to any of claims 1 and 5, wherein the processor (22) is configured to display at least a portion (28, 48) of the catheter (14) as illuminated differently by making the catheter appear (303, 313) to be more illuminated when it is closer to the viewer and appears more shaded when it is further away.

8. A method, comprising:
receiving an electroanatomical (EA) 3D map (20, 200) of a cardiac chamber, the map configured to be displayed on a display device (27) as a view-dependent orthographic projection;
receiving position information of a catheter (14) located within the cardiac chamber, from a catheter positioning system;
defining a reference point location (214) of the catheter displayed on the 3D map;
for any given viewing direction of the 3D map, defining the viewing direction as a ray superimposed on a vector from a virtual viewer to the reference point location (214);
calculating a view-dependent cardiac chamber distance (222) between a distal wall location along the ray and a proximal wall location along the ray;
calculating a location over the ray of the reference point location (214) relative to the cardiac chamber view-dependent distance (222), based on the position information; and
indicating the calculated reference point location (214) relative to the cardiac chamber view-dependent distance (222) to a user.

## Patentansprüche

1. System (10), umfassend:
eine Anzeigevorrichtung (27); und
einen Prozessor (22), der konfiguriert ist zum:
Empfangen einer elektroanatomischen (EA) 3D-Karte (20, 200) einer Herzkammer, wobei die Karte dazu konfiguriert ist, auf der Anzeigevorrichtung als eine ansichtsabhängige (2200, 2020) orthografische Projektion (202) angezeigt zu werden;
Empfangen von Positionsinformationen eines Katheters (14), der innerhalb der Herzkammer angeordnet ist, von einem Katheterpositionierungssystem;
Definieren einer Referenzpunktposition (214) des auf der 3D-Karte angezeigten Katheters;
Definieren der Betrachtungsrichtung als einen Strahl, der auf einen Vektor gelegt ist, von einem virtuellen Betrachter zu der Referenzpunktposition, für jede gegebene Betrachtungsrichtung der 3D-Karte;
Berechnen eines ansichtsabhängigen Herzkammerabstands (222) zwischen einer distalen Wandposition entlang des Strahls und einer proximalen Wandposition entlang des Strahls;
Berechnen einer Position über den Strahl der Referenzpunktposition (214) relativ zu dem ansichtsabhängigen Herzkammerabstand (222) basierend auf den Positionsinformationen; und
Angeben (217, 224, 303) der berechneten Referenzpunktposition (214) relativ zu dem ansichtsabhängigen Herzkammerabstand (222) für einen Benutzer.

2. System (10) nach Anspruch 1, wobei der Prozessor (22) dazu konfiguriert ist, die berechnete Referenzpunktposition (214) durch Anzeigen des berechneten Positionswerts über eine numerische Skala anzugeben, die durch den ansichtsabhängigen (2200, 2020) Herzkammerabstand (222) definiert ist.

3. System (10) nach Anspruch 1, wobei der Prozessor (22) dazu konfiguriert ist, die berechnete Referenzpunktposition (214) durch Anzeigen einer Hilfskarte (220) anzugeben, die aus einer orthografischen Ansicht aus einer Richtung orthogonal zu der gegebenen Betrachtungsrichtung erhalten wird.

4. System (10) nach Anspruch 1, wobei der Prozessor (22) dazu konfiguriert ist, die berechnete Referenzpunktposition (214) durch grafisches Codieren (217) eines Abschnitts (216) des Katheters (14) gemäß dem berechneten Referenzpositionswert (224) über eine numerische Skala anzugeben, die durch den ansichtsabhängigen Herzkammerabstand (222) definiert ist.

5. System (10) nach Anspruch 4, wobei der Prozessor (22) dazu konfiguriert ist, den Abschnitt (216) des Katheters (14) mit einer ersten Farbe grafisch zu codieren (217), wenn der berechnete Referenzpositionswert kleiner als die Hälfte des ansichtsabhängigen Herzkammerabstands (222) ist, und mit einer zweiten Farbe, wenn der berechnete Referenzpositionswert höher als die Hälfte des ansichtsabhängigen Herzkammerabstands (222) ist.

6. System (10) nach einem der Ansprüche 1 und 5, wobei der Prozessor (22) dazu konfiguriert ist, die berechnete Referenzpunktposition (214) anzugeben, indem zumindest ein Abschnitt (28, 48) des Katheters entsprechend seinem ansichtsabhängigen Abstand (222) vom Benutzer als unterschiedlich beleuchtet (303, 313) angezeigt wird.

7. System (10) nach einem der Ansprüche 1 und 5, wobei der Prozessor (22) dazu konfiguriert ist, zumindest einen Abschnitt (28, 48) des Katheters (14) als unterschiedlich beleuchtet anzuzeigen, indem bewirkt wird, dass der Katheter (303, 313) stärker beleuchtet erscheint, wenn er näher am Betrachter ist, und dunkler erscheint, wenn er weiter entfernt ist.

8. Verfahren, umfassend:
Empfangen einer elektroanatomischen (EA) 3D-Karte (20, 200) einer Herzkammer, wobei die Karte dazu konfiguriert ist, auf einer Anzeigevorrichtung (27) als eine ansichtsabhängige orthografische Projektion angezeigt zu werden;
Empfangen von Positionsinformationen eines Katheters (14), der innerhalb der Herzkammer angeordnet ist, von einem Katheterpositionierungssystem;
Definieren einer Referenzpunktposition (214) des auf der 3D-Karte angezeigten Katheters;
Definieren der Betrachtungsrichtung als einen Strahl, der auf einen Vektor gelegt ist, von einem virtuellen Betrachter zu der Referenzpunktposition (214) für jede gegebene Betrachtungsrichtung der 3D-Karte;
Berechnen eines ansichtsabhängigen Herzkammerabstands (222) zwischen einer distalen Wandposition entlang des Strahls und einer proximalen Wandposition entlang des Strahls;
Berechnen einer Position über den Strahl der Referenzpunktposition (214) relativ zu dem ansichtsabhängigen Herzkammerabstand (222) basierend auf den Positionsinformationen; und
Angeben der berechneten Referenzpunktposition (214) relativ zu dem ansichtsabhängigen Herzkammerabstand (222) für einen Benutzer.

## Revendications

1. Système (10), comprenant :
un dispositif d'affichage (27) ; et
un processeur (22) qui est configuré pour :
recevoir une carte 3D électroanatomique (EA) (20, 200) d'une chambre cardiaque, la carte étant configurée pour être affichée sur le dispositif d'affichage en tant que projection orthographique (202) dépendante de la vue (2200, 2020) ;
recevoir des informations de position d'un cathéter (14) localisé au sein de la chambre cardiaque, en provenance d'un système de positionnement de cathéter ;
définir une localisation de point de référence (214) du cathéter affiché sur la carte 3D ;
pour l'une quelconque direction de visualisation donnée de la carte 3D, définir la direction de visualisation en tant que rayon superposé sur un vecteur allant d'un observateur virtuel à la localisation de point de référence ;
calculer une distance (222) de chambre cardiaque dépendante de la vue entre une localisation de paroi distale le long du rayon et une localisation de paroi proximale le long du rayon ;
calculer une localisation sur le rayon de la localisation de point de référence (214) par rapport à la distance (222) de chambre cardiaque dépendante de la vue, en fonction des informations de position ; et
indiquer (217, 224, 303) la localisation de point de référence (214) calculée par rapport à la distance (222) de chambre cardiaque dépendante de la vue à un utilisateur.

2. Système (10) selon la revendication 1, dans lequel le processeur (22) est configuré pour indiquer la localisation de point de référence (214) calculée en affichant la valeur de localisation calculée sur une échelle numérique définie par la distance (222) de chambre cardiaque dépendante de la vue (2200, 2020).

3. Système (10) selon la revendication 1, dans lequel le processeur (22) est configuré pour indiquer la localisation de point de référence (214) calculée en affichant une carte auxiliaire (220) obtenue à partir d'une vue orthographique à partir d'une direction orthogonale à la direction de visualisation donnée.

4. Système (10) selon la revendication 1, dans lequel le processeur (22) est configuré pour indiquer la localisation de point de référence (214) calculée en encodant graphiquement (217) une partie (216) du cathéter (14) selon la valeur de localisation de référence calculée (224) sur une échelle numérique définie par la distance (222) de chambre cardiaque dépendante de la vue.

5. Système (10) selon la revendication 4, dans lequel le processeur (22) est configuré pour encoder graphiquement (217) la partie (216) du cathéter (14) par une première couleur si la valeur de localisation de référence calculée est plus petite que la moitié de la distance (222) de chambre cardiaque dépendante de la vue et par une seconde couleur si la valeur de localisation de référence calculée est supérieure à la moitié de la distance (222) de chambre cardiaque dépendante de la vue.

6. Système (10) selon l'une quelconque des revendications 1 et 5, dans lequel le processeur (22) est configuré pour indiquer la localisation de point de référence (214) calculée en affichant au moins une partie (28, 48) du cathéter illuminée différemment (303, 313) selon sa distance (222) dépendante de la vue par rapport à l'utilisateur.

7. Système (10) selon l'une quelconque des revendications 1 et 5, dans lequel le processeur (22) est configuré pour afficher au moins une partie (28, 48) du cathéter (14) illuminée différemment en faisant en sorte que le cathéter apparaisse (303, 313) plus illuminé lorsqu'il est plus près de l'observateur et apparaisse plus ombragé lorsqu'il est plus éloigné.

8. Procédé, comprenant :
la réception d'une carte 3D électroanatomique (EA) (20, 200) d'une chambre cardiaque, la carte étant configurée pour être affichée sur un dispositif d'affichage (27) en tant que projection orthographique dépendante de la vue ;
la réception d'informations de position d'un cathéter (14) localisé au sein de la chambre cardiaque, en provenance d'un système de positionnement de cathéter ;
la définition d'une localisation de point de référence (214) du cathéter affiché sur la carte 3D ;
pour l'une quelconque direction de visualisation donnée de la carte 3D, la définition de la direction de visualisation en tant que rayon superposé sur un vecteur allant d'un observateur virtuel à la localisation de point de référence (214) ;
le calcul d'une distance (222) de chambre cardiaque dépendante de la vue entre une localisation de paroi distale le long du rayon et une localisation de paroi proximale le long du rayon ;
le calcul d'une localisation sur le rayon de la localisation de point de référence (214) par rapport à la distance (222) de chambre cardiaque dépendante de la vue, en fonction des informations de position ; et
l'indication de la localisation de point de référence (214) calculée par rapport à la distance (222) de chambre cardiaque dépendante de la vue à un utilisateur.
